# EUROPEAN PATENT SPECIFICATION

(11) **EP 3 003 381 B1**
(45) Date of publication and mention of the grant of the patent: **13.09.2017**
(21) Application number: 14728567.0
(22) Date of filing: 05.06.2014
(51) Int. Cl.: A61K 9/14, A61K 47/10, A61P 35/00, A61K 31/437

(54) **PHARMACEUTICAL COMPOSITIONS**
PHARMAZEUTISCHE ZUSAMMENSETZUNGEN
COMPOSITIONS PHARMACEUTIQUES

(30) Priority: 06.06.2013 EP 13170754
(43) Date of publication of application: 13.04.2016
(73) Proprietor: Bayer Pharma Aktiengesellschaft, 13353 Berlin (DE)
(72) Inventor: SCHULZE, Volker, 16562 Hohen Neuendorf OT Bergfelde (DE); BRÜNING, Michael, 16552 Schildow (DE); STÖCKIGT, Detlef, Potsdam 14471 (DE)
(74) Representative: BIP Patents
(86) International application number: PCT/EP2014/061711
(87) International publication number: WO 2014/195408

(56) References cited:
- US-A1- 2012 328 611
- US-A1- 2013 121 994

## Description

The present invention relates to pharmaceutical compositions of (2*R*)-2-(4-fluorophenyl)-N-[4-(2-{[2-methoxy-4-(methylsulfonyl)phenyl]amino} [1,2,4]triazolo[1,5-*α*]pyridin-6-yl)phenyl]propanamide or a salt, hydrate or solvate thereof as described and defined herein, to methods of preparing said compositions, and to the use of the compositions for the treatment or prophylaxis of a disease.

### BACKGROUND OF THE INVENTION

The present invention relates to pharmaceutical compositions containing chemical compounds that inhibit Mps-1 (Monopolar Spindle 1) kinase (also known as Tyrosine Threonine Kinase, TTK).

Mps-1 is a dual specificity Ser/Thr kinase which plays a key role in the activation of the mitotic checkpoint (also known as spindle checkpoint, spindle assembly checkpoint) thereby ensuring proper chromosome segregation during mitosis [Abrieu A et al., Cell, 2001, 106, 83-93]. Every dividing cell has to ensure equal separation of the replicated chromosomes into the two daughter cells. Upon entry into mitosis, chromosomes are attached at their kinetochores to the microtubules of the spindle apparatus. The mitotic checkpoint is a surveillance mechanism that is active as long as unattached kinetochores are present and prevents mitotic cells from entering anaphase and thereby completing cell division with unattached chromosomes [Suijkerbuijk SJ and Kops GJ, Biochemica et Biophysica Acta, 2008, 1786, 24-31; Musacchio A and Salmon ED, Nat Rev Mol Cell Biol., 2007, 8, 379-93]. Once all kinetochores are attached in a correct amphitelic, *i.e.* bipolar, fashion with the mitotic spindle, the checkpoint is satisfied and the cell enters anaphase and proceeds through mitosis. The mitotic checkpoint consists of a complex network of a number of essential proteins, including members of the MAD (mitotic arrest deficient, MAD 1-3) and Bub (Budding uninhibited by benzimidazole, Bub 1-3) families, the motor protein CENP-E, Mps-1 kinase as well as other components, many of these being over-expressed in proliferating cells (*e.g.* cancer cells) and tissues [Yuan B et al., Clinical Cancer Research, 2006, 12, 405-10]. The essential role of Mps-1 kinase activity in mitotic checkpoint signalling has been shown by shRNA-silencing, chemical genetics as well as chemical inhibitors of Mps-1 kinase [Jelluma N et al., PLos ONE, 2008, 3, e2415; Jones MH et al., Current Biology, 2005, 15, 160-65; Dorer RK et al., Current Biology, 2005, 15, 1070-76; Schmidt M et al., EMBO Reports, 2005, 6, 866-72].
There is ample evidence linking reduced but incomplete mitotic checkpoint function with aneuploidy and tumorigenesis [Weaver BA and Cleveland DW, Cancer Research, 2007, 67, 10103-5; King RW, Biochimica et Biophysica Acta, 2008, 1786, 4-14]. In contrast, complete inhibition of the mitotic checkpoint has been recognised to result in severe chromosome missegregation and induction of apoptosis in tumour cells [Kops GJ et al., Nature Reviews Cancer, 2005, 5, 773-85; Schmidt M and Medema RH, Cell Cycle, 2006, 5, 159-63; Schmidt M and Bastians H, Drug Resistance Updates, 2007, 10, 162-81].

Therefore, mitotic checkpoint abrogation through pharmacological inhibition of Mps-1 kinase or other components of the mitotic checkpoint represents a new approach for the treatment of proliferative disorders including solid tumours such as carcinomas and sarcomas and leukaemias and lymphoid malignancies or other disorders associated with uncontrolled cellular proliferation.

WO2011/064328, WO2011/063907, WO2011/063908, and WO2013/087579A1 relate to [1,2,4]-triazolo-[1,5-α]-pyridines and their use for inhibition of Mps-1 kinase.

A preferred route of drug administration is through the oral cavity. This route provides the greatest comfort and convenience of dosing. The bioavailability achieved after oral administration is a measure for the potential usefulness of an oral dosage form of a drug. Bioavailability after oral application depends on several factors, such as solubility of the active in aqueous media, dose strength, dissolution of the dosage form, absorption throughout the gastrointestinal tract and first pass effect. Therefore solid pharmaceutical compositions for oral application containing an Mps-1 inhibitor, which result in improved dissolution, absorption and exposure in mammals, improved inter-patient variability, and overall improved efficacy in the clinic are desired.

### SUMMARY of the INVENTION

It has now been discovered that the target of improved efficacy, metabolic stability, dissolution, superior absorption and increased bioavailability and other needs, which will become apparent to one skilled in the art, are met by the present invention, which is described in detail below. The present invention provides a pharmaceutical composition as defined in claims. The present invention further relates to methods of preparing pharmaceutical compositions, and to the use of said pharmaceutical compositions for the treatment or prophylaxis of a disease.

### DETAILED DESCRIPTION of the INVENTION

The terms as mentioned in the present text have preferably the following meanings :
The term "halogen atom" or "halo-" is to be understood as meaning a fluorine, chlorine, bromine or iodine atom.

The term "C₁-C₆-alkyl" is to be understood as preferably meaning a linear or branched, saturated, monovalent hydrocarbon group having 1, 2, 3, 4, 5 or 6 carbon atoms, *e.g*. a methyl, ethyl, propyl, butyl, pentyl, hexyl, iso-propyl, iso-butyl, sec-butyl, tert-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl group, or an isomer thereof. Particularly, said group has 1, 2, 3 or 4 carbon atoms ("C₁-C₄-alkyl"), *e.g.* a methyl, ethyl, propyl, butyl, iso-propyl, iso-butyl, sec-butyl, tert-butyl group, more particularly 1, 2 or 3 carbon atoms ("C₁-C₃-alkyl"), *e.g*. a methyl, ethyl, n-propyl- or iso-propyl group.

The term "halo-C₁-C₆-alkyl" is to be understood as preferably meaning a linear or branched, saturated, monovalent hydrocarbon group in which the term "C₁-C₆-alkyl" is defined *supra,* and in which one or more of the hydrogen atoms is replaced, in identically or differently, by a halogen atom. Particularly, said halogen atom is F. Said halo-C₁-C₆-alkyl group is, for example, -CF₃, -CHF₂, -CH₂F, -CF₂CF₃, or -CH₂CF₃.

The term "C₁-C₆-alkoxy" is to be understood as preferably meaning a linear or branched, saturated, monovalent group of formula -O-(C₁-C₆-alkyl), in which the term "C₁-C₆-alkyl" is defined *supra, e.g.* a methoxy, ethoxy, n-propoxy, iso-propoxy, n-butoxy, iso-butoxy, tert-butoxy, sec-butoxy, pentoxy, iso-pentoxy, or n-hexoxy group, or an isomer thereof.

The term "halo-CrCe-alkoxy" is to be understood as preferably meaning a linear or branched, saturated, monovalent C₁-C₆-alkoxy group, as defined *supra,* in which one or more of the hydrogen atoms is replaced, in identically or differently, by a halogen atom. Particularly, said halogen atom is F. Said halo-C₁-C₆-alkoxy group is, for example, -OCF₃, -OCHF₂, -OCH₂F, -OCF₂CF₃, or - OCH₂CF₃.

The term "C₁-C₆-alkoxy-C₁-C₆-alkyl" is to be understood as preferably meaning a linear or branched, saturated, monovalent C₁-C₆-alkyl group, as defined *supra,* in which one or more of the hydrogen atoms is replaced, in identically or differently, by a C₁-C₆-alkoxy group, as defined *supra, e.g.* methoxyalkyl, ethoxyalkyl, propyloxyalkyl, iso-propoxyalkyl, butoxyalkyl, iso-butoxyalkyl, tert-butoxyalkyl, sec-butoxyalkyl, pentyloxyalkyl, iso-pentyloxyalkyl, hexyloxyalkyl group, or an isomer thereof.

The term "halo-C₁-C₆-alkoxy-C₁-C₆-alkyl" is to be understood as preferably meaning a linear or branched, saturated, monovalent C₁-C₆-alkoxy-C₁-C₆-alkyl group, as defined *supra,* in which one or more of the hydrogen atoms is replaced, in identically or differently, by a halogen atom. Particularly, said halogen atom is F. Said halo-C₁-C₆-alkoxy-C₁-C₆-alkyl group is, for example, - CH₂CH₂OCF₃, -CH₂CH₂OCHF₂, -CH₂CH₂OCH₂F, -CH₂CH₂OCF₂CF₃, or - CH₂CH₂OCH₂CF₃.

The term "C₂-C₆-alkenyl" is to be understood as preferably meaning a linear or branched, monovalent hydrocarbon group, which contains one or more double bonds, and which has 2, 3, 4, 5 or 6 carbon atoms, particularly 2 or 3 carbon atoms ("C₂-C₃-alkenyl"), it being understood that in the case in which said alkenyl group contains more than one double bond, then said double bonds may be isolated from, or conjugated with, each other. Said alkenyl group is, for example, a vinyl, allyl, (E)-2-methylvinyl, (Z)-2-methylvinyl, homoallyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (E)-3-methylpent-3-enyl, (Z)-3-methylpent-3-enyl, (E)-2-methylpent-3-enyl, (Z)-2-methylpent-3-enyl, (E)-1-methylpent-3-enyl, (Z)-1-methylpent-3-enyl, (E)-4-methylpent-2-enyl, (Z)-4-methylpent-2-enyl, (E)-3-methylpent-2-enyl, (Z)-3-methylpent-2-enyl, (E)-2-methylpent-2-enyl, (Z)-2-methylpent-2-enyl, (E)-1-methylpent-2-enyl, (Z)-1-methylpent-2-enyl, (E)-4-methylpent-1-enyl, (Z)-4-methylpent-1-enyl, (E)-3-methylpent-1-enyl, (Z)-3-methylpent-1-enyl, (E)-2-methylpent-1-enyl, (Z)-2-methylpent-1-enyl, (E)-1-methylpent-1-enyl, (Z)-1-methylpent-1-enyl, 3-ethylbut-3-enyl, 2-ethylbut-3-enyl, 1-ethylbut-3-enyl, (E)-3-ethylbut-2-enyl, (Z)-3-ethylbut-2-enyl, (E)-2-ethylbut-2-enyl, (Z)-2-ethylbut-2-enyl, (E)-1-ethylbut-2-enyl, (Z)-1-ethylbut-2-enyl, (E)-3-ethylbut-1-enyl, (Z)-3-ethylbut-1-enyl, 2-ethylbut-1-enyl, (E)-1-ethylbut-1-enyl, (Z)-1-ethylbut-1-enyl, 2-propylprop-2-enyl, 1-propylprop-2-enyl, 2-isopropylprop-2-enyl, 1-isopropylprop-2-enyl, (E)-2-propylprop-1-enyl, (Z)-2-propylprop-1-enyl, (E)-1-propylprop-1-enyl, (Z)-1-propylprop-1-enyl, (E)-2-isopropylprop-1-enyl, (Z)-2-isopropylprop-1-enyl, (E)-1-isopropylprop-1-enyl, (Z)-1-isopropylprop-1-enyl, (E)-3,3-dimethylprop-1-enyl, (Z)-3,3-dimethylprop-1-enyl, 1-(1,1-dimethylethyl)ethenyl, buta-1,3-dienyl, penta-1,4-dienyl, hexa-1,5-dienyl, or methylhexadienyl group. Particularly, said group is vinyl or allyl.

The term "C₂-C₆-alkynyl" is to be understood as preferably meaning a linear or branched, monovalent hydrocarbon group which contains one or more triple bonds, and which contains 2, 3, 4, 5 or 6 carbon atoms, particularly 2 or 3 carbon atoms ("C₂-C₃-alkynyl"). Said C₂-C₆-alkynyl group is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-inyl, hex-3-inyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethyl-but-3-inyl, 1,1-dimethylbut-3-ynyl, 1,1-dimethylbut-2-ynyl, or 3,3-dimethyl-but-1-ynyl group. Particularly, said alkynyl group is ethynyl, prop-1-ynyl, or prop-2-inyl.

The term "C₃-C₆-cycloalkyl" is to be understood as preferably meaning a saturated, monovalent, mono-, or bicyclic hydrocarbon ring which contains 3, 4, 5 or 6 carbon atoms. Said C₃-C₆-cycloalkyl group is for example, a monocyclic hydrocarbon ring, *e.g*. a cyclopropyl, cyclobutyl, cyclopentyl, or cyclohexyl or a bicyclic hydrocarbon ring. Said cycloalkyl ring can optionally contain one or more double bonds *e.g*. cycloalkenyl, such as a cyclopropenyl, cyclobutenyl, cyclopentenyl or cyclohexenyl group, wherein the bond between said ring with the rest of the molecule may be to any carbon atom of said ring, be it saturated or unsaturated.

The term "heterocyclic ring", as used in the term "4-, 5- or 6- membered heterocyclic ring", or "4- to 6-membered heterocyclic ring" or "4- to 5-membered heterocyclic ring", for example, as used in the definition of compounds of general formula (I) as defined herein, is to be understood as meaning a saturated monocyclic nitrogen atom-containing ring, said nitrogen atom being the point of attachment of said heterocyclic ring with the rest of the molecule. Said nitrogen atom-containing ring optionally further contains 1 or 2 heteroatom-containing groups selected from O and C(=O). Particularly, without being limited thereto, said nitrogen atom-containing ring can be a 4-membered ring, such as an azetidinyl ring, for example, or a 5-membered ring, such as a pyrrolidinyl ring or oxazolidinonyl ring, for example, or a 6-membered ring, such as a piperidinyl or morpholinyl ring, for example ; it being reiterated that any of the above-mentioned nitrogen atom-containing rings can further contain 1 or 2 heteroatom-containing groups selected from O and C(=O).
As mentioned *supra,* said nitrogen atom-containing ring can be partially unsaturated, *i.e.* it can contain one or more double bonds, such as, without being limited thereto, a 2,5-dihydro-1H-pyrrolyl ring, for example.

The term "3- to 10-membered heterocycloalkyl" is to be understood as meaning a saturated monovalent, monocyclic hydrocarbon ring which contains 2, 3, 4, 5, 6, 7, 8, or 9 carbon atoms, and one or more heteroatom-containing groups selected from C(=O), O, S, S(=O), S(=O)₂, NH, NR", wherein R" represents a C₁-C₆-alkyl, C₃-C₆-cycloalkyl, -C(=O)- (C₁-C₆-alkyl) or -C(=O)-(C₁-C₆-cycloalkyl). Particularly, said ring can contain 2, 3, 4, or 5 carbon atoms, and one or more of the above-mentioned heteroatom-containing groups (a "3- to 6-membered heterocycloalkyl"), more particularly said ring can contain 4 or 5 carbon atoms, and one or more of the above-mentioned heteroatom-containing groups (a "5- to 6-membered heterocycloalkyl"). Said heterocycloalkyl ring is for example, a monocyclic heterocycloalkyl ring such as an oxyranyl, oxetanyl, aziridinyl, azetidinyl, tetrahydrofuranyl, imidazolidinyl, pyrazolidinyl, tetrahydropyranyl, piperidinyl, morpholinyl, dithianyl, thiomorpholinyl, piperazinyl, trithianyl, or chinuclidinyl group.

The term "aryl" is to be understood as meaning a monovalent, aromatic or partially aromatic, mono-, or bi- or tricyclic hydrocarbon ring having 6, 7, 8, 9, 10, 11, 12, 13 or 14 carbon atoms (a "C₆-C₁₄-aryl" group), particularly a ring having 6 carbon atoms (a "C₆-aryl" group), *e.g*. a phenyl group, or a biphenyl group, or a ring having 9 carbon atoms (a "C₉-aryl" group), *e.g*. an indanyl or indenyl group, or a ring having 10 carbon atoms (a "C₁₀-aryl" group), *e.g*. a tetralinyl, dihydronaphthyl, or naphthyl group, or a ring having 13 carbon atoms, (a "C₁₃-aryl" group), *e.g*. a fluorenyl group, or a ring having 14 carbon atoms, (a "C₁₄-aryl" group), *e.g*. an anthranyl group.

The term "heteroaryl" is understood as preferably meaning a monovalent, aromatic, mono- or bicyclic aromatic ring system having 5, 6, 7, 8, 9, 10, 11, 12, 13 or 14 ring atoms (a "5- to 14-membered heteroaryl" group), particularly 5 or 6 or 9 or 10 atoms, and which contains at least one heteroatom which may be identical or different, said heteroatom being such as oxygen, nitrogen or sulfur, and can be monocyclic, bicyclic, or tricyclic, and in addition in each case can be benzocondensed. Particularly, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl *etc.,* and benzo derivatives thereof, such as, for example, benzofuranyl, benzothienyl, benzoxazolyl, benzisoxazolyl, benzimidazolyl, benzotriazolyl, indazolyl, indolyl, isoindolyl, *etc*.; or pyridyl, pyridazinyl, pyrimidinyl, pyrazinyl, triazinyl, *etc.,* and benzo derivatives thereof, such as, for example, quinolinyl, quinazolinyl, isoquinolinyl, *etc*.; or azocinyl, indolizinyl, purinyl, *etc.,* and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, *etc.* More particularly, heteroaryl is selected from pyridyl, benzofuranyl, benzisoxazolyl, indazolyl, quinazolinyl, thienyl, quinolinyl, benzothienyl, pyrazolyl, or furanyl.

The term "alkylene" is understood as preferably meaning an optionally substituted hydrocarbon chain (or "tether") having 1, 2, 3, 4, 5, 6, 7 or 8 carbon atoms, *i.e.* an optionally substituted -CH₂- ("methylene" or "single membered tether" or, for example -C(CH₃)₂-), -CH₂-CH₂- ("ethylene", "dimethylene", or "two-membered tether", for example -C(CH₃)₂-C(CH₃)₂-), - CH₂-CH₂-CH₂- ("propylene", "trimethylene", or "three-membered tether", for example -CH₂-C(H)(CH₃)-CH₂-, -CH₂-C(CH₃)₂-CH₂-), -CH₂-CH₂-CH₂-CH₂-("butylene", "tetramethylene", or "four-membered tether"), -CH₂-CH₂-CH₂-CH₂-CH₂- ("pentylene", "pentamethylene" or "five-membered ether"), or -CH₂-CH₂-CH₂-CH₂-CH₂-CH₂- ("hexylene", "hexamethylene", or six-membered tether") group. Particularly, said alkylene tether has 1, 2, 3, 4, or 5 carbon atoms, more particularly 1 or 2 carbon atoms.

The term "C₁-C₆", as used throughout this text, *e.g*. in the context of the definition of "C₁-C₆-alkyl", "C₁-C₆-haloalkyl", "C₁-C₆-alkoxy", or "C₁-C₆-haloalkoxy" is to be understood as meaning an alkyl group having a finite number of carbon atoms of 1 to 6, *i.e.* 1, 2, 3, 4, 5, or 6 carbon atoms. It is to be understood further that said term "C₁-C₆" is to be interpreted as any sub-range comprised therein, *e.g.* C₁-C₆, C₂-C₅, C₃-C₄ , C₁-C₂, C₁-C₃ , C₁-C₄ , C₁-C₅, C₁-C₆; particularly C₁-C₂, C₁-C₃ , C₁-C₄ , C₁-C₅, C₁-C₆ ; more particularly C₁-C₄; in the case of "C₁-C₆-haloalkyl" or "C₁-C₆-haloalkoxy" even more particularly C₁-C₂.

Similarly, as used herein, the term "C₂-C₆", as used throughout this text, *e.g*. in the context of the definitions of "C₂-C₆-alkenyl" and "C₂-C₆-alkynyl", is to be understood as meaning an alkenyl group or an alkynyl group having a finite number of carbon atoms of 2 to 6, *i.e.* 2, 3, 4, 5, or 6 carbon atoms. It is to be understood further that said term "C₂-C₆" is to be interpreted as any sub-range comprised therein, *e.g*. C₂-C₆, C₃-C₅, C₃-C₄ , C₂-C₃, C₂-C₄, C₂-C₅; particularly C₂-C₃.

Further, as used herein, the term "C₃-C₆", as used throughout this text, *e.g*. in the context of the definition of "C₃-C₆-cycloalkyl", is to be understood as meaning a cycloalkyl group having a finite number of carbon atoms of 3 to 6, *i.e.* 3, 4, 5 or 6 carbon atoms. It is to be understood further that said term "C₃-C₆" is to be interpreted as any sub-range comprised therein, *e.g*. C₃-C₆ , C₄-C₅, C₃-C₅, C₃-C₄, C₄-C₆, C₅-C₆; particularly C₃-C₆.

As used herein, the term "leaving group" refers to an atom or a group of atoms that is displaced in a chemical reaction as stable species taking with it the bonding electrons. Preferably, a leaving group is selected from the group comprising: halo, in particular chloro, bromo or iodo, methanesulfonyloxy, p-toluenesulfonyloxy, trifluoromethanesulfonyloxy, nonafluorobutanesulfonyloxy, (4-bromo-benzene)sulfonyloxy, (4-nitrobenzene)sulfonyloxy, (2-nitro-benzene)-sulfonyloxy, (4-isopropylbenzene)sulfonyloxy, (2,4,6-tri-isopropyl-benzene)-sulfonyloxy, (2,4,6-trimethyl-benzene)sulfonyloxy, (4-tertbutyl-benzene)sulfonyloxy, benzenesulfonyloxy, and (4-methoxy-benzene)sulfonyloxy.

As used herein, the term "PG¹" refers to a protecting group for hydroxy groups e.g. a TMS group or TBDPS group as decribed for example in T.W. Greene and P.G.M. Wuts in Protective Groups in Organic Synthesis, 3rd edition, Wiley 1999 (TMS = trimethylsilyl, TBDPS = tert-butyldiphenylsilyl).

As used herein, the term "PG²" refers to a protecting group for amino groups e.g. a Boc group as descibed for example in T.W. Greene and P.G.M. Wuts in Protective Groups in Organic Synthesis, 3rd edition, Wiley 1999 (Boc = *tert-*butyloxycarbonyl).

As used herein, the term "one or more times", *e.g*. in the definition of the substituents of the compounds of the general formulae of the present invention, is understood as meaning "one, two, three, four or five times, particularly one, two, three or four times, more particularly one, two or three times, even more particularly one or two times".

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g*., chiral HPLC columns), with or without conventional derivatisation, optimally chosen to maximise the separation of the enantiomers. Suitable chiral HPLC columns are manufactured by Diacel, *e.g*., Chiracel OD and Chiracel OJ among many others, all routinely selectable. Enzymatic separations, with or without derivatisation, are also useful. The optically active compounds of this invention can likewise be obtained by chiral syntheses utilizing optically active starting materials.

In order to limit different types of isomers from each other reference is made to IUPAC Rules Section E (Pure Appl Chem 45, 11-30, 1976).

The invention also includes all suitable isotopic variations of a compound of the invention. An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen, oxygen, phosphorus, sulphur, fluorine, chlorine, bromine and iodine, such as ²H (deuterium), ³H (tritium), ¹¹C, ¹³C, ¹⁴C, ¹⁵N, ¹⁷O, ¹⁸O, ³²P, ³³P, ³³S_{,} ³⁴S, ³⁵S, ³⁶S, ¹⁸F, ³⁶Cl, ⁸²Br, ¹²³I, ¹²⁴I, ¹²⁹I and ¹³¹I, respectively. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain therapeutic advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

Further, the compounds of the present invention may exist as tautomers. For example, any compound of the present invention which contains a pyrazole moiety as a heteroaryl group for example can exist as a 1H tautomer, or a 2H tautomer, or even a mixture in any amount of the two tautomers, or a triazole moiety for example can exist as a 1H tautomer, a 2H tautomer, or a 4H tautomer, or even a mixture in any amount of said 1H, 2H and 4H tautomers, *viz.* :

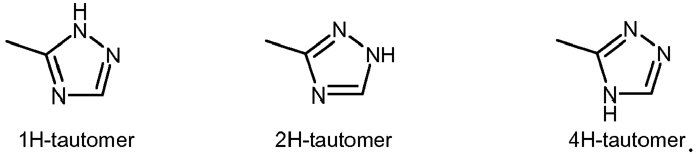

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

### Compound A

The pharmaceutical composition of the present invention comprises the compound A which is (2*R*)-2-(4-fluorophenyl)-N-[4-(2-{[2-methoxy-4-(methylsulfonyl)phenyl]amino}[1,2,4]triazolo[1,5-*α*]pyridin-6-yl)phenyl]propanamide. The compound A or a salt, hydrate or solvate thereof, may be present in the pharmaceutical composition according to the invention partly or completely in amorphous form and/or thermodynamically metastable crystal modification. The pharmaceutical composition according to the invention preferably comprises the active ingredient in amorphous form and/or in the form of a metastable crystal modification in an amount of at least 50%, particularly preferably more than 50%, in particular at least 90%, based on the total amount of active ingredient present.

### Matrix B

A pharmaceutical composition of the present invention comprises a matrix B. The term "matrix", as used herein, refers to polymeric excipients, non-polymeric excipients, and combinations thereof, capable of dissolving or dispersing a compound A.

The task of the matrix is to keep the compound A in a water-soluble state or in a state in which the compound A is soluble in physiological media, respectively. It is supposed that a compound A dissolved or dispersed in a matrix according to the present invention is kept in an amorphous form and/or a metastable crystal modification and is prevented from forming a thermodynamically stable crystalline modification: as long as the largest proportion of the amount of the compound A present in a pharmaceutical composition does not form a thermodynamically stable crystalline modification but remains in an amourphous form or in a metastable crystal modification, it is sufficiently water-soluble (soluble in physiological media) to act as an active ingredient in a pharmaceutical composition.

### PEG/PVP based compositions

In a first aspect, the matrix B consists of
i. 70% to 100% by weight of polyethylene glycol having an average molecular weight of from 100 to 800;
ii. 0% to 30% by weight of polyvinylpyrrolidone;
iii. 0% to 10% by weight of water; and
iv. 0% to 5% by weight of one or more pharmaceutically acceptable excipients.

It is obvious, that the sum of the percentages by weight of components i, ii and iii has to be 100%. So, when for example the matrix B consists of 100% by weight of polyethyleneglycol, it contains 0% polyvinylpyrrolidone, 0% water and 0% of a further pharmaceutically acceptable excipient. When for example the the matrix B consists of 80% by weight of polyethyleneglycol, and 20% by weight of polyvinylpyrrolidone, it contains 0% water and and 0% of a further pharmaceutically acceptable excipient.

In a preferred embodiment, the matrix B consists of
i. 70% to 90% by weight of polyethylene glycol having an average molecular weight of from 100 to 800;
ii. 10% to 30% by weight of polyvinylpyrrolidone;
iii. 0% to 10% by weight of water; and
iv. 0% to 5% by weight of one or more pharmaceutically acceptable excipients.

In another preferred embodiment, the matrix B consists of
i. 80% to 95% by weight of polyethylene glycol having an average molecular weight of from 100 to 800;
ii. 0% to 20% by weight of polyvinylpyrrolidone; and
v. 0% to 10% by weight of water; and
vi. 0% to 3% by weight of one or more pharmaceutically acceptable excipients.

In another preferred embodiment, the matrix B consists of
i. 80% to 95% by weight of polyethylene glycol having an average molecular weight of from 100 to 800;
ii. 0% to 20% by weight of polyvinylpyrrolidone; and
iii. 0% to 3 % by weight of one or more pharmaceutically acceptable excipients.

In another preferred embodiment, the matrix B consists of
i. 75% to 85% by weight of polyethylene glycol having an average molecular weight of from 100 to 800;
ii. 15% to 25% by weight of polyvinylpyrrolidone.

In another preferred embodiment, the matrix B consists of
i. 80% by weight of polyethylene glycol having an average molecular weight of from 100 to 800;
ii. 20% by weight of polyvinylpyrrolidone.

In another preferred embodiment, the matrix B consists of
i. 97% to 100% by weight of polyethylene glycol having an average molecular weight of from 100 to 800;
ii. 0% to 3 % by weight of one or more pharmaceutically acceptable excipients.

In another preferred embodiment, the matrix B consists of 100% polyethylene glycol having an average molecular weight of from 100 to 800.

In another preferred embodiment, the polyethylene glycol has an average molecular weight of from 200 to 600.

In another preferred embodiment, the polyethylene glycol has an average molecular weight of from 300 to 500.

In another preferred embodiment, the polyethylene glycol has an average molecular weight of from 350 to 550.

In another preferred embodiment, the polyethylene glycol has an average molecular weight of 400.

Historically, it was difficult to determine the molecular weight of polyvinylpyrrolidone. Thus, the K-value was adopted to classify the various molecular weights of PVP polymers. The K-value, a function of the average degree of polymerization and the intrinsic viscosity of the polymer, is calculated from kinematic viscosity of an aqueous polymer solution. Products of different K-values are commercially available, e.g. K12, K17, K25, K30, and K90. BASF markets these products under the brand name Kollidon^{®}.

The polyvinylpyrrolidone used in the pharmaceutical composition of the present invention has a K-value between 23 and 27.

In a preferred embodiment, the polyvinylpyrrolidone has a K-value between 24 and 26.

In another preferred embodiment, the polyvinylpyrrolidone is PVP K25.

According to the first aspect of the present invention, the pharmaceutical composition comprises a compound A of general formula (I), *supra,* or a salt, hydrate or solvate thereof, and a matrix B as defined *supra;* wherein the weight ratio of the compound A of formula (I) calculated as a solvent-free base to the matrix B is smaller than 20 : 80.

In a preferred embodiment, the weight ratio of the compound A of formula (I) calculated as a solvent-free base to the matrix B is between 1 : 99 to 20 : 80.

In another preferred embodiment, the weight ratio of the compound A of formula (I) calculated as a solvent-free base to the matrix B is between 1 : 99 to 15 : 85.

In another preferred embodiment, the weight ratio of the compound A of formula (I) calculated as a solvent-free base to the matrix B is between 1 : 99 to 10 : 90.

In another preferred embodiment, the weight ratio of the compound A of formula (I) calculated as a solvent-free base to the matrix B is between 1 : 99 to 5 : 95.

In another preferred embodiment, the weight ratio of the compound A of formula (I) calculated as a solvent-free base to the matrix B is between 1 : 99 to 3 : 97.

The matrix B comprises polyethylene glycol; it optionally comprises polyvinylpyrrolidone (up to 20% by weight) and water (up to 10% by weight). The aim of the matrix B is to keep the active ingredient in a state in which it is water-soluble or soluble in physiological media, respectively. So, the matrix B is considered to have a stabilizing effect on the amourphous or meta-crystalline form of the active ingredient (compound A).

In a particularly preferred embodiment, the pharmaceutical composition consists of 2 to 3 weight percent of a compound A of general formula (I), *supra,* or a salt, hydrate or solvate thereof, 76 to 79 weight percent of PEG 400 and 18 to 22 weight percent of PVP, whith the proviso that the sum of the weight percentages of the ingredients is 100.

It is possible to add one or more further pharmaceutically acceptable excipients to the matrix B without losing the stabilizing effect of the matrix B.

Such pharmaceutically acceptable excipients can be e.g. antioxidants, buffering agents, colorants, flavorants, sweetening agents, etc.

Pharmaceutically acceptable excipients are well known to the one of ordinary skills in the art and are described *inter alia* in the following references, each of which is incorporated herein by reference: Powell, M.F. et al., "Compendium of Excipients for Parenteral Formulations" PDA Journal of Pharmaceutical Science Et Technology 1998, 52(5), 238-311 ; Strickley, R.G "Parenteral Formulations of Small Molecule Therapeutics Marketed in the United States (1999)-Part-1" PDA Journal of Pharmaceutical Science Et Technology 1999, 53(6), 324-349 ; and Nema, S. et al., "Excipients and Their Use in Injectable Products" PDA Journal of Pharmaceutical Science Et Technology 1997, 51(4), 166-171.

The maximum amount of further pharmaceutically acceptable excipients that can be added to the matrix B without losing the stabilizing effect can be determined by routine experiments. It is considered that usually an amount of further pharmaceutically acceptable excipients of up to 5 % by weight has no or only a negligible effect on the stabilizing effect of the matrix B.

### Co-precipitates

In a second aspect, the matrix B consists of
i. 40% to 60% by weight of polyvinylpyrrolidone;
ii. 40% to 60% by weight of croscarmellose sodium; and
iii. 0% to 5 % by weight of one or more pharmaceutically acceptable excipients.

It is obvious, that the sum of the percentages by weight of components i, ii and iii has to be 100%. So, when for example the matrix B consists of 40% by weight of polyvinylpyrrolidone, and 60% by weight of croscarmellose sodium, it must contain 0% of a further pharmaceutically acceptable excipient.

In a preferred embodiment, the matrix B consists of
i. 40% to 50% by weight of polyvinylpyrrolidone;
ii. 50% to 60% by weight of croscarmellose sodium; and
iii. 0% to 4% by weight of one or more pharmaceutically acceptable excipients.

In another preferred embodiment, the matrix B consists of
i. 45% to 49% by weight of polyvinylpyrrolidone;
ii. 51% to 55% by weight of croscarmellose sodium; and
iii. 0% to 3% by weight of one or more pharmaceutically acceptable excipients.

In another preferred embodiment, the matrix B consists of
i. 46% to 48% by weight of polyvinylpyrrolidone;
ii. 52% to 54% by weight of croscarmellose sodium; and
iii. 0% to 2% by weight of one or more pharmaceutically acceptable excipients.

According to the second aspect of the present invention, the pharmaceutical composition comprises a compound A of general formula (I), *supra,* or a salt, hydrate or solvate thereof, and a matrix B as defined *supra;* wherein the weight ratio of the compound A of formula (I) calculated as a solvent-free base to the matrix B smaller than 20 : 80.

In a preferred embodiment, the weight ratio of the compound A of formula (I) calculated as a solvent-free base to the matrix B is between 1 : 99 to 20 : 80.

In another preferred embodiment, the weight ratio of the compound A of formula (I) calculated as a solvent-free base to the matrix B is between 1 : 99 to 15 : 85.

In another preferred embodiment, the weight ratio of the compound A of formula (I) calculated as a solvent-free base to the matrix B is between 1 : 99 to 12 : 88.

In another preferred embodiment, the weight ratio of the compound A of formula (I) calculated as a solvent-free base to the matrix B is between 1 : 99 to 10 : 80.

In another preferred embodiment, the weight ratio of the compound A of formula (I) calculated as a solvent-free base to the matrix B is between 1 : 99 to 9 : 91.

In another preferred embodiment, the weight ratio of the compound A of formula (I) calculated as a solvent-free base to the matrix B is between 2 : 98 to 8 : 92.

In another preferred embodiment, the weight ratio of the compound A of formula (I) calculated as a solvent-free base to the matrix B is between 3 : 97 to 7 : 93.

In another preferred embodiment, the weight ratio of the compound A of formula (I) calculated as a solvent-free base to the matrix B is between 4 : 96 to 6 : 94.

The matrix B comprises polyvinylpyrrolidone and croscarmellose sodium. The aim of the matrix B is to keep the active ingredient in a state in which it is water-soluble or soluble in physiological media, respectively. So, the matrix B is considered to have a stabilizing effect on the amourphous or meta-crystalline form of the active ingredient (compound A).

It is possible to add one or more further pharmaceutically acceptable excipients to the matrix B without losing the stabilizing effect of the matrix B.

Such pharmaceutically acceptable excipients can be e.g. antioxidants, buffering agents, colorants, flavorants, sweetening agents, etc.

Pharmaceutically acceptable excipients are well known to the one of ordinary skills in the art and are described *inter alia* in the following references, each of which is incorporated herein by reference: Powell, M.F. et al., "Compendium of Excipients for Parenteral Formulations" PDA Journal of Pharmaceutical Science Et Technology 1998, 52(5), 238-311 ; Strickley, R.G "Parenteral Formulations of Small Molecule Therapeutics Marketed in the United States (1999)-Part-1" PDA Journal of Pharmaceutical Science Et Technology 1999, 53(6), 324-349 ; and Nema, S. et al., "Excipients and Their Use in Injectable Products" PDA Journal of Pharmaceutical Science Et Technology 1997, 51(4), 166-171.

The maximum amount of further pharmaceutically acceptable excipients that can be added to the matrix B without losing the stabilizing effect can be determined by routine experiments. It is considered that usually an amount of further pharmaceutically acceptable excipients of up to 5% by weight has no or only a negligible effect on the stabilizing effect if the matrix B.

### PEG melts

In a third aspect, the matrix B consists of 75% to 100% by weight of polyethylene glycol having an average molecular weight of from 4000 to 8000 and 25% to 0% of polyvinylpyrrolidone, preferably Kollidon 25.

In a preferred embodiment, the matrix B consists of 95% to 100% by weight of polyethylene glycol having an average molecular weight of from 4000 to 8000.

In another preferred embodiment, the matrix B consists of 97% to 100% by weight of polyethylene glycol.

In another preferred embodiment, the polyethylene glycol has an average molecular weight of from 4500 to 7500.

In another preferred embodiment, the polyethylene glycol has an average molecular weight of from 5000 to 7000.

In another preferred embodiment, the polyethylene glycol has an average molecular weight of from 5500 to 6500.

In another preferred embodiment, the polyethylene glycol has an average molecular weight of 6000.

According to the third aspect of the present invention, the pharmaceutical composition comprises a compound A of general formula (I), *supra,* or a salt, hydrate or solvate thereof, and a matrix B as defined *supra*; wherein the weight ratio of the compound A of formula (I) calculated as a solvent-free base to the matrix B is smaller than 30 : 70.

In a preferred embodiment, the weight ratio of the compound A of formula (I) calculated as a solvent-free base to the matrix B is between 1 : 99 to 30 : 70.

In another preferred embodiment, the weight ratio of the compound A of formula (I) calculated as a solvent-free base to the matrix B is between 1 : 99 to 25 : 75.

In another preferred embodiment, the weight ratio of the compound A of formula (I) calculated as a solvent-free base to the matrix B is between 2 : 98 to 24 : 76.

In another preferred embodiment, the weight ratio of the compound A of formula (I) calculated as a solvent-free base to the matrix B is between 3 : 97 to 23 : 77.

In another preferred embodiment, the weight ratio of the compound A of formula (I) calculated as a solvent-free base to the matrix B is between 4 : 96 to 22 : 78.

In another preferred embodiment, the weight ratio of the compound A of formula (I) calculated as a solvent-free base to the matrix B is between 5 : 95 to 21 : 79.

In another preferred embodiment, the weight ratio of the compound A of formula (I) calculated as a solvent-free base to the matrix B is between 5 : 95 to 20 : 80.

In another preferred embodiment, the weight ratio of the compound A of formula (I) calculated as a solvent-free base to the matrix B is between 6 : 94 to 17 : 83.

In another preferred embodiment, the weight ratio of the compound A of formula (I) calculated as a solvent-free base to the matrix B is between 7 : 93 to 16 : 84.

In another preferred embodiment, the weight ratio of the compound A of formula (I) calculated as a solvent-free base to the matrix B is between 7 : 93 to 15 : 85.

The matrix B mainly consists of polyethylene glycol. The aim of the matrix B is to keep the active ingredient in a state in which it is water-soluble or soluble in physiological media, respectively. So, the matrix B is considered to have a stabilizing effect on the amourphous or meta-crystalline form of the active ingredient (compound A).

It is possible to add one or more further pharmaceutically acceptable excipients to the matrix B without losing the stabilizing effect of the matrix B.

Such pharmaceutically acceptable excipients can be e.g. antioxidants, buffering agents, colorants, flavorants, sweetening agents, etc..

Pharmaceutically acceptable excipients are well known to the one of ordinary skills in the art and are described *inter alia* in the following references, each of which is incorporated herein by reference: Powell, M.F. et al., "Compendium of Excipients for Parenteral Formulations" PDA Journal of Pharmaceutical Science Et Technology 1998, 52(5), 238-311 ; Strickley, R.G "Parenteral Formulations of Small Molecule Therapeutics Marketed in the United States (1999)-Part-1" PDA Journal of Pharmaceutical Science Et Technology 1999, 53(6), 324-349 ; and Nema, S. et al., "Excipients and Their Use in Injectable Products" PDA Journal of Pharmaceutical Science Et Technology 1997, 51(4), 166-171.

The maximum amount of further pharmaceutically acceptable excipients that can be added to the matrix B without losing the stabilizing effect can be determined by routine experiments. It is considered that usually an amount of further pharmaceutically acceptable excipients of up to 5% has no or only a negligible effect.

### Pharmaceutical composition

The pharmaceutical compositions of the invention are prepared according to methods known to the art for the manufacture of solid dispersions, such as fusion/melt technology, hot melt extrusion, coprecipitation, and the like.

The pharmaceutical compositions will be utilized to achieve the desired pharmacological effect by oral administration to a patient in need thereof, and will be advantageous to a conventional formulation in terms of drug release, bioavailability, inter-patient variability and/or efficacy in mammals.

A patient, for the purpose of this invention, is a mammal, including a human, in need of treatment for the particular condition or disease, including prophylactic treatment.

For oral administration, the solid dispersion described herein can be formulated into solid or liquid preparations such as powders, granules, pellets, tablets, capsules, dragees, chewable tablets, effervescent tablets, dispersible tablets, troches, lozenges, melts, solutions, suspensions, or emulsions, and may be prepared according to methods known to the art for the manufacture of pharmaceutical compositions. For this purpose the solid dispersion may be compounded with conventional excipients, for example binders, fillers, lubricants, disintegrants, solvents, surfactants, emulsifiers, thickeners and stabilizers, glidants and lubricants, coating materials as well as sweeteners, flavoring and coloring agents.

It is believed that one skilled in the art, utilizing the preceding information, ean utilize the present invention to its fullest extent. The oral formulation of the compound A of formula (I) or a salt, hydrate or solvate thereof refers to a wide range of dosages such as 1 mg, 10 mg, 100 mg, or even 1 g daily dosing and beyond. This would be accomplished, for example, by modifying the composition and size of the tablet or capsule, and/or by administering multiple tablets or capsules per day to the patient in need thereof. Alternatively, the solid dispersion formulation may also be dosed in forms such as powders, granules, chewable, efferveseent or dispersible tablets, or by dispersions of any adequate solid formulation in a suitable liquid prior to use, for example if the optimal dose regimen was no longer consistent with a feasible tablet or capsule size.

### Method of treating hyper-proliferative disorders

The present invention also retates to a method for using a new oral pharmaceutical composition of the present invention to treat mammalian hyper-proliferative disorders, including cancer. This method comprises administering the pharmaceutical composition in the form of a solid dispersion to a mammal in need thereof, including a human, an amount which is effective to treat the disorder.

The term "hyperproliferative disorders" and/or "cancer" not only refers to solid tumors, such as cancers of the breast, respiratory tract, brain, reproductive organs, digestive tract, urinary tract, eye, liver, skin, head and neck, thyroid, parathyroid and their distant metastases, but also includes lymphomas, sarcomas, and leukemias.

Examples of breast cancer include, but are not limited to invasive ductal carcinoma, invasive lobular carcinoma, ductal carcinoma *in situ,* and lobular carcinoma *in situ.*

Examples of cancers of the respiratory tract include, but are not limited to small-cell and non-small-cell lung carcmoma, as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal, and vulvar cancer, as well as sarcoma of the uterus.

Tumors of the digestive tract include, but are not limited to anal, colon, colorectal, esophageal, gallbladder, gastric, pancreatic, rectal, small intestine, and salivary gland cancers.

Tumors of the urinary tract include, but are not limited to bladder, penile, kidney, renal pelvis, ureter, and urethral cancers.

Eye cancers include, but are not l1mlted to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma,

Skin cancers include, but are not limited to squamous cell carcinoma, Kaposi 's sarcoma, malignant melanoma, Merkel cell skin cancer, and non-melanoma skin cancer.

Head-and-neck cancers include, but are not limited to laryngeal / hypopharyngeal / nasopharyngeal / oropharyngeal cancer, and lip and oral cavity cancer.

Lymphomas include, but are not limited to AIDS-related lymphoma, non-Hodgkin's lymphoma, cutaneous T-cell lymphoma, Hodgkin's disease, and lymphoma of the central nervous system.

Sarcomas include, but are not limited to sarcoma of the soft tissue, fibrosarcoma, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well charactenzed in humans, but also exist with a similar etiology in other mammals, such as canines and felines, and can be treated by administering the pharmaceutical compositions of the present invention.

The total amount of the active ingredient (compound A) to be administered via the oral route using the pharmaceutical composition of the present invention will generally range from about 0.01 mg/kg to about 50 mg/kg body weight per day. Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the pharmaceutical compositions of this invention can readily be determined by those skilled in the art. The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

The pharmaceutical compositions of this invention can be administered as the sole agent or in combination with one or more other therapies where the combination causes no unacceptable adverse effects. For example, they can be combined with cytotoxic agents, signal transduction inhibitors, or with other anti-cancer agents or therapies, as well as with admixtures and combinations thereof.

### EXPERIMENTAL SECTION

### Preparation of compounds of general formula (I)

Substituted triazolopyridine compounds of gerenal formula (I) were prepared according to the methods described in WO2013/087579A1 and WO2014/009219(A1).

### Biological data

Assays for determining the biological data of compounds of gerenal formula (I) are described in WO2013/087579A1.

The compounds of general formula (I) are characterized by the following attributes (see WO2013/087579A1 for more details):
- The IC₅₀ determined in an Mps-1 kinase assay with a concentration of 10 µM ATP is lower than or equal to 1 nM.
- The IC₅₀ determined in an Mps-1 kinase assay with a concentration of 2 mM ATP is lower than 10 nM. The IC₅₀ of preferred compounds is even lower than 5 nM. The IC₅₀ of more preferred compounds is even lower than 3 nM. The IC₅₀ of most preferred compounds is even lower than 2 nM.
- The maximum oral bioavailability (Fₘₐₓ) in rat (determined by means of rat liver microsomes) is higher than 50%. The Fₘₐₓ of preferred compounds is even higher than 70%. The Fₘₐₓ of more preferred compounds is even higher than 80%.
- The maximum oral bioavailability (Fₘₐₓ) in dog (determined by means of dog liver microsomes) is higher than 45%. The Fₘₐₓ of preferred compounds is even higher than 52%. The Fₘₐₓ of more preferred compounds is even higher than 70%.
- The maximum oral bioavailability (Fₘₐₓ) in human (determined by means of human liver microsomes) is higher than 45%. The Fₘₐₓ of preferred compounds is even higher than 60%. The Fₘₐₓ of more preferred compounds is even higher than 85%.
- The IC₅₀ determined in a HeLa cell proliferation assay is lower than 600 nM. The IC₅₀ of preferred compounds is even lower than 400 nM. The IC₅₀ of more preferred compounds is even lower than 200 nM. The IC₅₀ of most preferred compounds is even lower than 100 nM.

### Pharmaceutical compositions of the present invention

### PEG/PVP based compositions

### Examples:

PEG400 and PVP (Kollidon® 25) were added together in the ratio of 80:20 (w/w) and mixed with a magnetic stirrer until a clear solution was obtained. One part of (2*R*)-2-(4-fluorophenyl)-*N*-[4-(2-{[2-methoxy-4-(methylsulfonyl)-phenyl]amino}[1,2,4]triazolo[1,5-*a*]pyridin-6-yl)phenyl]propanamide was added to 20-200 parts of the PEG400 / PVP solution. The mixture was stirred with a magnetic stirrer until a clear solution was obtained.

Solubility of (2*R*)-2-(4-nuorophenyl)-*N*-[4-(2-{[2-methoxy-4-(methybulfonyl)-phenyl]amino}[1,2,4]triazolo[1,5-*a*]pyridin-6-yl)phenyl)propanamide in a PEG400 based LSF (Liquide Service Formulation: PEG400/Tween 80/L-Menthol 97:2,5:0,5) amounts to appr. 25 mg/ml. A nine months stability study at 25 and 40°C in PEG400 based LSF was performed showing no significant time dependant instability.
Solubility of (2*R*)-2-(4-fluorophenyl)-*N*-[4-(2-{[2-methoxy-4-(methylsulfonyl)-phenyl])amino}[1,2,4]triazolo[1,5-*a*]pyridin-6-yl)phenyl]propanamide in PEG400 / PVP / water 8:1:1 and in PEG400 / PVP 8:2 amounts to appr. 25 mg/ml.

### Co-precipitates

### Example:

(2*R*)-2-(4-fluorophenyl)-*N*-[4-(2-{[2-methoxy-4-(methylsulfonyl)phenyl]-amino}[1,2,4]triazolo[1,5-*a*]pyridin-6-yl)phenyl]propanamide was dissolved in a 80:20 mixture of acetone/ethanol (w/w). To one part of the solved (2R)-2-(4-fluorophenyl)-*N*-[4-(2-{[2-methoxy-4-(methylsulfonyl)phenyl]-amino}[1,2,4]triazolo[1,5-*a*]pyridin-6-yl)phenyl]propanamide, four parts PVP and 5 parts AcDiSol were added. The mixture was stirred with a magnetic stirrer until a clear solution was obtained. Using a rotary vacuum evaporator the solvent was removed at app. 70°C. The dry residue was removed from the evaporation flask and pestled.

### PEG melts

### Example:

Nine parts of PEG6000 were melted in an aluminium cup (70° to 90°C). One part of (2*R*)-2-(4-fluorophenyl)-N-[4-(2-{[2-methoxy-4-(methylsulfonyl)phenyl]-amino}[1,2,4]triazolo[1,5-*a*]pyridin-6-yl)phenyl]propanamide was added to the PEG 6000 melt. The mixture was stirred with a glass stick at approximately 90°C. After complete dissolution of (2*R*)-2-(4-fluorophenyl)-*N*-[4-(2-{[2-methoxy-4-(methylsulfonyl)phenyl]amino}[1,2,4]triazolo[1,5-*a*]pyridin-6-yl)phenyl]propanamide, the melt was cooled down by embedding the cup in a ice-water/NaCl mixture for several minutes. The solidified melt was pestled. (2*R*)-2-(4-fluorophenyl)-*N*-[4-(2-{[2-methoxy-4-(methylsulfonyl)phenyl]-amino}[1,2,4]triazolo[1,5-*a*]pyridin-6-yl)phenyl]propanamide is stable in PEG6000 melt with 20% drug load for minimally two months at room temperature.

### Oral bioavailability

### General

Tylose^{®} MH 300 (Sigma), hereinafter referred to as Tylose, is a methylhydroxyethyl cellose ether which is a water-soluble non-ionic polymer used in materials to provide water retention, binding, thickening, film forming and colloid properties. Tylose^{®} has to be stirred and swelled for app. 12 h in pure water (w/v), resulting in a viscous mixture. The drug substance has to be grinded in the viscous mixture, resulting in a Tylose^{®} suspension of the drug substance.

Ac-Di-Sol (croscarmellose sodium) is a crosslinked sodium carboxymethyl cellulose.

Solutol HS 15 (BASF) is a nonionic solubilizer and emulsifying agent obtained by reacting 15 moles of ethylene oxide with 1 mole of 12-hydroxy stearic acid. The main application is as nonionic solubiliser for manufacturing of aqueous parenteral preparations.

Compound A used in the oral bioavailability studies was (2R)-2-(4-fluorophenyl)-*N*-[4-(2-{[2-methoxy-4-(methylsulfonyl)phenyl]-amino}[1,2,4]triazolo[1,5-*a*]pyridin-6-yl)phenyl]propanamide.

### Studies

In order to investigate the relative bioavailability of compound A, different studies were conducted in Wistar rats (220-290 g; 6-8 weeks old). Three of them will be reported in the following, among them administration of aqueous Tylose suspension, LSF-based PEG400 solution and salt suspensions in water. Each of them is referenced to the formulation of the free base in PEG400/EtOH/Solutol HS 15 (70/5/25).

In a first study microcrystalline aqueous suspension of 0.5% Tylose^{®} (w/v), the reference solution (PEG400/EtOH/Solutol = 70/5/25), and an aqueous suspension of Ac-Di-Sol/PVP co-precipitate have been administered to female Wistar rats (n = 3 each) at 1.0 mg/kg.

Approximately 0.4 mL of whole blood was collected via an indwelling jugular catheter. The blood samples were centrifuged (approx. 5 min.) in order to obtain plasma which was then transferred to the appropriately labelled vials and stored frozen (-20°C) until analysis for parent drug. Plasma samples were analysed via LC/MS/MS for parent drug concentration and pharmacokinetic parameters using KinEx. The results are summarized in Table A.

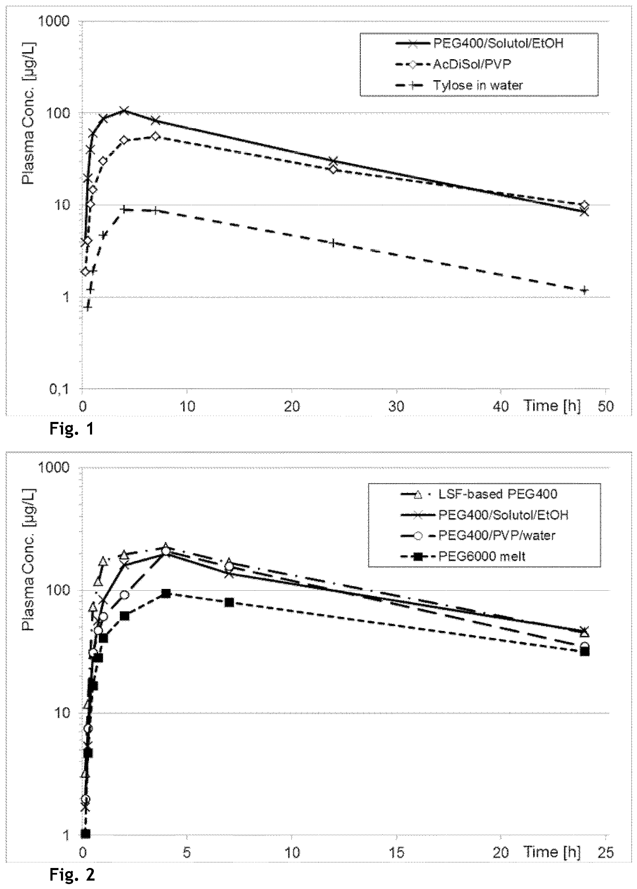

Fig. 1 shows the plasma concentration after p.o. administration of Tylose suspension (0.5% in aq. NaCl), reference solution (PEG400/Solutol/EtOH=70/25/5), and co-precipitate suspension (AcDiSol/PVP/compound A = 50/45/5) to female rats at a dose of 1.0 mg/kg of compound A.

Plasma concentration-time courses run almost parallel after administration of these formulations (Fig. 1). In Tylose suspension slightly retarded absorption was observed and in co-precipitate slightly increased half-life. The peak plasma concentration was observed 7, 4 and 4 h after administration for Tylose suspension, reference solution and co-precipitate, respectively. C_{max,norm} (0.018, 0.11 and 0.068 kg/L) and AUC(0-tₗₐₛₜ)ₙₒᵣₘ (0.35, 1.9 and 1.4 kg h/L) differ across theses formulations with corresponding relative bioavailabilities amounting to 18%, 100% and 74%, respectively.

In order to find suitable alternative formulations, 1.9 mg/kg of the compound A was administered to female Wistar rats (n = 3) in a reference solution (PEG400/Solutol/EtOH = 70/25/5), a solution of PEG400/PVP/water (80/10/10), a PEG6000/compound A (70/30) melt suspended in water, and a solution of LSF-based PEG400. The results are summarized in Table B.

Fig. 2 shows the plasma concentration after p.o. administration of 1.9 mg/kg of the compound A to female Wistar rats (n = 3) in a reference solution (PEG400/Solutol/EtOH = 70/25/5), a solution of PEG400/PVP/water (80/10/10), a suspension of PEG6000/compound A (70/30) melt suspended in water, and a solution of LSF-based PEG400.

Plasma concentration-time courses run almost parallel after administration of these formulations (Fig. 2). A slightly accelerated absorption is indicated by the LSF-based PEG400 formulation with peak plasma concentration after 2 h in contrast to 4 h for all other formulations. C_{max,norm} (0.11, 0.12, 0.052 and 0.15 kg/L) and AUC(0-tₗₐₛₜ)ₙₒᵣₘ (1.3, 1.3, 0.73 and 1.5 kg h/L) differ across the formulations used with corresponding relative bioavailabilities amounting to 100%, 56.2% and 115% for PEG400/PVP/water, PEG6000 melt and LSF-based PEG400, respectively.

**Table A: Pharmacokinetic parameters after p.o. administration of Tylose suspension (0.5% in aqueous NaCl), reference solution (PEG400/Solutol/EtOH=70/25/5) and co-precipitate suspension (AcDiSol/PVP/compound A = 50/45/5) to female rats at a dose of 1.0 mg/kg of the compound A.**

| Dose | mg/kg | 1.0 | 1.0 | 1.0 |
|---|---|---|---|---|
| Formulation | | PEG400 70% + Solutol 25% + EtOH 5% (Sol) | Tylose 0.5% + aq. NaCl 99.5% (Susp) | AcDiSol 50% + PVP 45% + Drug 5% (Susp) |
| V_{administered} | mL/kg | 2.0 | 2.0 | 2.0 |
| AUC | mg.h/L | 2.0 | 0.23 | 1.8 |
| AUC(0-tₗₐₛₜ) | mg.h/L | 1.9 | 0.20 | 1.4 |
| AUCₙₒᵣₘ | kg·h/L | 2 ·/: 1.1 | 0.4 ·/: 1.4 | 1.8 ·/: 1.2 |
| AUC(0-tₗₐₛₜ)ₙₒᵣₘ | kg·h/L | 1.9 ·/: 1.2 | 0.35 ·/: 1.3 | 1.4 ·/: 1.2 |
| F | % | 100 | 18 | 74 |
| Cₘₐₓ | mg/L | 0.11 | 0.010 | 0.068 |
| C_{max,norm} | kg/L | 0.11 ·/: 1.2 | 0.018 ·/: 1.1 | 0.068 ·/: 1.7 |
| t_{max,median} | h | 4.0 | 7.0 | 4.0 |
| t_{1/2} | h | 13 ·/: 1.1 | 14 ·/: 1.1 | 19 ·/: 1.9 |

**Table B: Pharmacokinetic parameters after p.o. administration of 1.9 mg/kg of the compound A to female Wistar rats (n = 3) in a reference solution (PEG400/Solutol/EtOH = 70/25/5, V_{administered} = 2 mL/kg), a solution of PEG400/PVP/water (80/10/10), a PEG6000/compound of A (70/30) melt suspended in water and a solution of LSF-based PEG400.**

| Dose | mg/kg | 1.9 | 1.9 | 1.9 | 1.9 |
|---|---|---|---|---|---|
| Formulation | | PEG400 70% + Solutol 25% + EtOH 5% (Sol) | PEG400 80% + PVP 10% + Water 10% (Sol) | PEG6000 70% + Drug Subtance 30% (Susp in water) | LSF-based PEG400 100% (Sol) |
| AUC | mg·h/L | 3.1 | 2.8 | 2.1 | 3.5 |
| AUC(0-tₗₐₛₜ) | mg·h/L | 2.4 | 2.4 | 1.4 | 2.9 |
| AUCₙₒᵣₘ | kg·h/L | 1.6 ·/: 1.2 | 1.5 ·/: 1.8 | 1.1 ·/: 1.6 | 1.8 ·/: 1.4 |
| AUC(0-tₗₐₛₜ)ₙₒᵣₘ | kg·h/L | 1.3 ·/: 1.2 | 1.3 ·/: 1.8 | 0.73 ·/: 1.8 | 1.5 ·/: 1.4 |
| Fᵣₑₗ | % | 100 | 100 | 56.2 | 115 |
| Cₘₐₓ | mg/L | 0.20 | 0.23 | 0.099 | 0.28 |
| C_{max,norm} | kg/L | 0.11 ·/: 1.4 | 0.12 ·/: 1.8 | 0.052 ·/: 2.2 | 0.15 ·/: 1.7 |
| t_{max,median} | h | 4.0 | 4.0 | 4.0 | 2.0 |
| t_{1/2} | h | 10 ·/: 1.1 | 7.8 ·/: 1.1 | 14 ·/: 1.6 | 8.8 ·/:1.1 |

## Claims

1. A pharmaceutical composition comprising:
a) a compound A, which is (2*R*)-2-(4-fluorophenyl)-*N*-[4-(2-{[2-methoxy-4-(methylsulfonyl)phenyl]amino}[1,2,4]triazolo[1,5-*a*]pyridin-6-yl)phenyl]propanamide,
or a salt, hydrate or solvate thereof;
and
b1) a matrix B1, said matrix B1 consisting of
i. 70% to 100% by weight of polyethylene glycol having an average molecular weight of from 100 to 800;
ii. 0% to 30% by weight of polyvinylpyrrolidone; and
iii. 0% to 10% by weight of water; and
iv. 0% to 5% by weight of one or more pharmaceutically acceptable excipients;
wherein the weight ratio of the compound A calculated as a solvent-free base to the matrix B1 is smaller than 20 : 80;
or
b2) a matrix B2, said matrix B2 consisting of
i. 40% to 60% by weight of polyvinylpyrrolidone
ii. 40% to 60% by weight of croscarmellose sodium; and
iii. 0% to 5% by weight of one or more pharmaceutically acceptable excipients;
wherein the weight ratio of the compound A calculated as a solvent-free base to the matrix B2 is smaller than 20 : 80;
or
b3) a matrix B3, said matrix B3 consisting of
i. 95% to 100% by weight of a polyethylene glycol having an average molecular weight of from 4000 to 8000; and
ii. 0% to 5% by weight of one or more pharmaceutically acceptable excipients;
wherein the weight ratio of the compound A calculated as a solvent-free base to the matrix B3 is smaller than 30 : 70.

2. A pharmaceutical composition according to claim 1, comprising a matrix B1; wherein the matrix B1 consists of
i. 80% to 95% by weight of polyethylene glycol;
ii. 0% to 20% by weight of polyvinylpyrrolidone; and
i. 0% to 10% by weight of water; and
ii. 0% to 3% by weight of one or more pharmaceutically acceptable excipients;
wherein the polyethylene glycol has an average molecular weight of from 300 to 500; and wherein the weight ratio of the compound A calculated as a solvent-free base to the matrix B1 is between 1 : 99 to 9 : 91.

3. A pharmaceutical composition according to claim 1, comprising a matrix B2; wherein the matrix B2 consists of
i. 45% to 49% by weight of polyvinylpyrrolidone;
ii. 51% to 55% by weight of croscarmellose sodium; and
iii. 0% to 3% by weight of one or more pharmaceutically acceptable excipients;
wherein the weight ratio of the compound A calculated as a solvent-free base to the matrix B2 is between 1 : 99 to 9 : 91.

4. A pharmaceutical composition according to claim 1, comprising a matrix B3; wherein the matrix B3 consists of 97% to 100% by weight of polyethylene glycol; wherein the polyethylene glycol has an average molecular weight of from 5500 to 6500; and wherein the weight ratio of the compound A calculated as a solvent-free base to the matrix B is between 5 : 95 to 20 : 80.

5. A pharmaceutical composition according to claim 1, consisting of 2% to 3% by weight of a compound A, or a salt, hydrate or solvate thereof, 76% to 79% by weight of polyethylene glycol having an average molecular weight of from 300 to 500, and 18% to 22% by weight of polyvinylpyrrolidone, with the proviso that the sum of the weight percentages of the ingredients is 100.

6. A pharmaceutical composition according to any one of claims 1 to 5 for use in the treatment or prophylaxis of a disease, wherein said disease is a disease of uncontrolled cell growth, proliferation and/or survival, an inappropriate cellular immune response, or an inappropriate cellular inflammatory response, particularly in which the uncontrolled cell growth, proliferation and/or survival, inappropriate cellular immune response, or inappropriate cellular inflammatory response is mediated by Mps-1, more particularly in which the disease of uncontrolled cell growth, proliferation and/or survival, inappropriate cellular immune response, or inappropriate cellular inflammatory response is a haemotological tumour, a solid tumour and/or metastases thereof, *e.g*. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

## Patentansprüche

1. Pharmazeutische Zusammensetzung, umfassend:
eine Verbindung A, bei der es sich um (2*R*)-2-(4-fluorphenyl)-*N*-[4-(2-{[2-methoxy-4-(methylsulfonyl)phenyl]amino}[1,2,4]triazolo[1,5-*a*]pyridin-6-yl)phenyl]propanamid handelt, oder ein Salz, Hydrat oder Solvat davon,
und
b1) eine Matrix B1, wobei die Matrix B1 aus
i. 70 Ges.-% bis 100 Gew.-% Polyethylenglykol mit einem durchschnittlichen Molekulargewicht von 100 bis 800,
ii. 0 Gew.-% bis 30 Gew.-% Polyvinylpyrrolidon und
iii. 0 Gew.-% bis 10 Gew.-% Wasser und
iv. 0 Ges.-% bis 5 Ges.-% an einem oder mehreren pharmazeutisch unbedenklichen Exzipienten
besteht, wobei das Gewichtsverhältnis von Verbindung A, berechnet als lösungsmittelfreie Base, zu der Matrix B1 kleiner als 20:80 ist, oder
b2) eine Matrix B2, wobei die Matrix B2 aus
i. 40 Gew.-% bis 60 Gew.-% Polyvinylpyrrolidon,
ii. 40 Gew.-% bis 60 Ges.-% Croscarmellose-Natrium und
iii. 0 Ges.-% bis 5 Ges.-% an einem oder mehreren pharmazeutisch unbedenklichen Exzipienten besteht, wobei das Gewichtsverhältnis von Verbindung A, berechnet als lösungsmittelfreie Base, zu der Matrix B2 kleiner als 20:80 ist,
oder
b3) eine Matrix B3, wobei die Matrix B3 aus
i. 95 Gew.-% bis 100 Gew.-% eines Polyethylenglykols mit einem durchschnittlichen Molekulargewicht von 4000 bis 8000 und
ii. 0 Gew.-% bis 5 Gew.-% an einem oder mehreren pharmazeutisch unbedenklichen Exzipienten
besteht, wobei das Gewichtsverhältnis von Verbindung A, berechnet als lösungsmittelfreie Base, zu der Matrix B3 kleiner als 30:70 ist.

2. Pharmazeutische Zusammensetzung nach Anspruch 1, umfassend eine Matrix B1, wobei die Matrix B1 aus
i. 80 Gew.-% bis 95 Gew.-% Polyethylenglykol,
ii. 0 Gew.-% bis 20 Gew.-% Polyvinylpyrrolidon und
i. 0 Gew.-% bis 10 Gew.-% Wasser und
ii. 0 Gew.-% bis 3 Gew.-% an einem oder mehreren pharmazeutisch unbedenklichen Exzipienten,
besteht, wobei das Polyethylenglykol ein durchschnittliches Molekulargewicht von 300 bis 500 hat und wobei das Gewichtsverhältnis von Verbindung A, berechnet als lösungsmittelfreie Base, zu der Matrix B1 zwischen 1:99 und 9:91 liegt.

3. Pharmazeutische Zusammensetzung nach Anspruch 1, umfassend eine Matrix B2, wobei die Matrix B2 aus
i. 45 Gew.-% bis 49 Gew.-% Polyvinylpyrrolidon,
ii. 51 Gew.-% bis 55 Gew.-% Croscarmellose-Natrium und
iii. 0 Gew.-% bis 3 Gew.-% an einem oder mehreren pharmazeutisch unbedenklichen Exzipienten,
besteht, wobei das Gewichtsverhältnis von Verbindung A, berechnet als lösungsmittelfreie Base, zu der Matrix B2 zwischen 1:99 und 9:91 liegt.

4. Pharmazeutische Zusammensetzung nach Anspruch 1, umfassend eine Matrix B3, wobei die Matrix B3 aus 97 Gew.-% bis 100 Gew.-% Polyethylenglykol besteht, wobei das Polyethylenglykol ein durchschnittliches Molekulargewicht von 5500 bis 6500 hat und wobei das Gewichtsverhältnis von Verbindung A, berechnet als lösungsmittelfreie Base, zu der Matrix B zwischen 5:95 und 20:80 liegt.

5. Pharmazeutische Zusammensetzung nach Anspruch 1, bestehend aus 2 Gew.-% bis 3 Gew.-% einer Verbindung A oder eines Salzes, Hydrats oder Solvats davon, 76 Gew.-% bis 79 Gew.-% Polyethylenglykol mit einem durchschnittlichen Molekulargewicht von 300 bis 500 und 18 Gew.-% bis 22 Gew.-% Polyvinylpyrrolidon, mit der Maßgabe, dass die Summe der Gewichtsprozente der Inhaltsstoffe 100 beträgt.

6. Pharmazeutische Zusammensetzung nach einem der Ansprüche 1 bis 5 zur Verwendung bei der Behandlung oder Prophylaxe einer Krankheit, wobei es sich bei der Krankheit um eine Krankheit mit unkontrolliertem Zellwachstum, einer unkontrollierten Zellproliferation und/oder unkontrolliertem Zellüberleben, eine unangemessene zelluläre Immunreaktion oder eine unangemessene zelluläre Entzündungsreaktion handelt, wobei das unkontrollierte Zellwachstum, die unkontrollierte Zellproliferation und/oder das unkontrollierte Zellüberleben, die unangemessene zelluläre Immunreaktion bzw. die unangemessene zelluläre Entzündungsreaktion insbesondere durch Mps-1 vermittelt wird, wobei es sich bei der Krankheit mit unkontrolliertem Zellwachstum, unkontrollierter Zellproliferation und/oder unkontrolliertem Zellüberleben, der unangemessenen zellulären Immunreaktion bzw. der unangemessenen zellulären Entzündungsreaktion ganz insbesondere um einen hämatologischen Tumor, einen festen Tumor und/oder Metastasen davon, z.B. Leukämien und myelodysplastisches Syndrom, maligne Lymphome, Kopf- und Halstumore einschließlich Hirntumore und Hirnmetastasen, Tumore des Thorax einschließlich nichtkleinzelligen und kleinzelligen Lungentumoren, Tumoren des Magen-Darm-Trakts, endokrine Tumore, Brust- und andere gynäkologische Tumore, urologische Tumore einschließlich Nieren-, Blasen- und Prostatatumoren, Hauttumore und Sarkome und/oder Metastasen davon handelt.

## Revendications

1. Composition pharmaceutique comprenant :
a) un composé A, qui est le (2R)-2-(4-fluorophényl)-N-[4-(2-{[2-méthoxy-4-(méthylsulfonyl)phényl]amino}[1,2,4]triazolo[1,5-a]pyridin-6-yl)phényl]propanamide,
ou sel, hydrate ou solvate de celui-ci ;
et
b1) une matrice B1, ladite matrice B1 étant constituée de
i. 70 % à 100 % en masse de polyéthylène glycol de masse moléculaire moyenne comprise entre 100 et 800 ;
ii. 0 % à 30 % en masse de polyvinylpyrrolidone ; et
iii. 0 % à 10 % en masse d'eau ; et
iv. 0 % à 5 % en masse d'un ou de plusieurs excipients pharmaceutiquement acceptables ;
où le rapport massique du composé A calculé sous forme de base exempte de solvants sur la matrice B1 est inférieur à 20 : 80 ;
ou
b2) une matrice B2, ladite matrice B2 étant constituée de
i. 40 % à 60 % en masse de polyvinylpyrrolidone
ii. 40 % à 60 % en masse de croscarmellose sodium ; et
iii. 0 % à 5 % en masse d'un ou de plusieurs excipients pharmaceutiquement acceptables ;
où le rapport massique du composé A calculé sous forme de base exempte de solvants sur la matrice B2 est inférieur à 20 : 80 ;
ou
b3) une matrice B3, ladite matrice B3 étant constituée de
i. 95 % à 100 % en masse de polyéthylène glycol de masse moléculaire moyenne comprise entre 4000 et 8000 ;
et
ii. 0 % à 5 % en masse d'un ou de plusieurs excipients pharmaceutiquement acceptables ;
où le rapport massique du composé A calculé sous forme de base exempte de solvants sur la matrice B3 est inférieur à 30 : 70.

2. Composition pharmaceutique selon la revendication 1, comprenant une matrice B1 ; où la matrice B1 est constituée de
i. 80 % à 95 % en masse de polyéthylène glycol ;
ii. 0 % à 20 % en masse de polyvinylpyrrolidone ; et
i. 0 % à 10 % en masse d'eau ; et
ii. 0 % à 3 % en masse d'un ou de plusieurs excipients pharmaceutiquement acceptables ;
où le polyéthylène glycol présente une masse moléculaire moyenne comprise entre 300 et 500 ; et
où le rapport massique du composé A calculé sous forme de base exempte de solvants sur la matrice B1 est compris entre 1 : 99 et 9 : 91.

3. Composition pharmaceutique selon la revendication 1, comprenant une matrice B2 ; où la matrice B2 est constituée de
i. 45 % à 49 % en masse de polyvinylpyrrolidone ;
ii. 51 % à 55 % en masse de croscarmellose sodium ; et
iii. 0 % à 3 % en masse d'un ou de plusieurs excipients pharmaceutiquement acceptables ;
où le rapport massique du composé A calculé sous forme de base exempte de solvants sur la matrice B2 est compris entre 1 : 99 et 9 : 91.

4. Composition pharmaceutique selon la revendication 1, comprenant une matrice B3 ; où la matrice B3 est constituée de 97 % à 100 % en masse de polyéthylène glycol ; où le polyéthylène glycol présente une masse moléculaire moyenne comprise entre 5500 et 6500 ; et où le rapport massique du composé A calculé sous forme de base exempte de solvants sur la matrice B est compris entre 5 : 95 et 20 : 80.

5. Composition pharmaceutique selon la revendication 1, constituée de 2 % à 3 % en masse d'un composé A, ou d'un sel, hydrate ou solvate de celui-ci, 76 % à 79 % en masse de polyéthylène glycol présentant une masse moléculaire moyenne comprise entre 300 et 500, et 18 % à 22 % en masse de polyvinylpyrrolidone, à la condition que la somme des pourcentages massiques des composants soit égale à 100.

6. Composition pharmaceutique selon l'une quelconque des revendications 1 à 5 pour utilisation dans le traitement prophylactique ou thérapeutique d'une maladie, où ladite maladie est une maladie de croissance, prolifération et/ou survie cellulaire incontrôlée, une réponse immunitaire cellulaire inappropriée, ou une réponse inflammatoire cellulaire inappropriée, en particulier dans laquelle la croissance, prolifération et/ou survie cellulaire incontrôlée, la réponse immunitaire cellulaire inappropriée, ou la réponse inflammatoire cellulaire inappropriée fait intervenir Mps-1, plus particulièrement dans laquelle la maladie de croissance, prolifération et/ou survie cellulaire incontrôlée, la réponse immunitaire cellulaire inappropriée, ou la réponse inflammatoire cellulaire inappropriée est une tumeur hématologique, une tumeur solide et/ou leurs métastases, par exemple les leucémies et le syndrome myélodysplasique, les lymphomes malins, les tumeurs de la tête et du cou y compris les tumeurs cérébrales et les métastases cérébrales, les tumeurs du thorax y compris les tumeurs pulmonaires à petites cellules et non à petites cellules, les tumeurs gastro-intestinales, les tumeurs endocrines, les tumeurs mammaires et autres tumeurs gynécologiques, les tumeurs urologiques y compris les tumeurs rénales, vésicales et prostatiques, les tumeurs cutanées et les sarcomes, et/ou leurs métastases.
